# EUROPEAN PATENT APPLICATION

(11) **EP 3 881 838 A1**
(43) Date of publication of application: **22.09.2021**
(21) Application number: 19883499.6
(22) Date of filing: 13.11.2019
(51) Int. Cl.: A61K 31/167, A61P 25/04, A61K 9/14, A61K 9/20, A61K 9/32, A61K 47/32

(54) **BONDING AGENT**

(30) Priority: 13.11.2018 JP 2018213350
(71) Applicant: Japan Vam & Poval Co., Ltd., Sakai-shi, Osaka 592-8331 (JP)
(72) Inventor: KAWADA, Shotaro, Sakai-shi, Osaka 592-8331 (JP); KAWANISHI, Masatoshi, Sakai-shi, Osaka 592-8331 (JP)
(74) Representative: Lederer & Keller Patentanwälte Partnerschaft mbB
(86) International application number: PCT/JP2019/044505
(87) International publication number: WO 2020/100932

(57) **Abstract**

A novel binder is provided. The binder comprises a polyvinyl alcohol-based polymer which has an average saponification value of 85.0 mol% to 89.0 mol% as measured according to JIS K6726 and meets requirement (A): a fluid prepared by adding 130.0 mL of 1-propanol to 100.0 g of a 5.0% by mass aqueous solution of the polyvinyl alcohol-based polymer has a transparency of 80.0% or less at 20°C; and/or requirement (B) : a supernatant from a fluid prepared by adding 230.0 mL of 1-propanol to 100.0 g of a 5.0% by mass aqueous solution of the polyvinyl alcohol-based polymer has a concentration of 0.65% by mass or more at 20°C.

## Description

### TECHNICAL FIELD

The present invention relates to a binder etc.

### BACKGROUND ART

Dosage forms of pharmaceutical products and health foods for oral ingestion of active ingredients are granules, tablets, powders, capsules, etc. For these applications, powder materials including medicinal substances and active ingredients are used, but in most cases, medicinal substances and active ingredients have a small particle diameter, drifting and dusting problems, and poor flowability and handleability.

For these reasons, such medicinal substances and active ingredients are usually subjected to granulation. Particularly, granulation in manufacturing pharmaceutical products etc. is also intended for improvement in tableting property, content uniformity, dissolution etc.

Patent Literature 1 (WO 2016/72179) discloses a composition comprising a specific polyvinyl alcohol-based polymer (hereinafter referred to as a PVA-based polymer) for film coating of solid preparations such as tablets.

### CITATION LIST

### Patent Literature

Patent Literature 1: WO 2016/72179

### SUMMARY OF INVENTION

### TECHNICAL PROBLEM

An object of the present invention is to provide a novel binder and the like.

### SOLUTION TO PROBLEM

As described in Patent Literature 1, coating techniques (film coating) for tablets etc. using PVA-based polymers are known. Such coating is intended for masking, oxygen insulation, moisture-proofing, product appearance improvement, etc. and is totally different in purpose and application from binders.

For example, for binders used for production of granulated materials from powder particles, binding strength and viscosity should be enough to efficiently bind powder particles together. In contrast, for film coating of tablets, it is necessary to avoid adhesion between tablets as much as possible from the viewpoint of productivity etc., and viscosity and tackiness are to be improved rather than desired. In fact, Patent Literature 1 states that the use of a specific PVA-based polymer can solve the problem of tackiness and achieves less adhesion between tablets.

Under such circumstances, the present inventors eagerly sought to use PVA-based polymers as binders. As a result, the present inventors found that a specific PVA-based polymer is useful as a binder based on an idea that is totally different from that of Patent Literature 1 etc., which is intended for film coating. The present inventors further conducted a great deal of examination and then completed the present invention.

That is, the present invention relates to the following.
[1] A binder comprising a polyvinyl alcohol-based polymer, wherein the polyvinyl alcohol-based polymer has an average saponification value of 85.0 mol% to 89.0 mol% as measured according to JIS K6726 and meets requirement (A): a fluid prepared by adding 130.0 mL of 1-propanol to 100.0 g of a 5.0% by mass aqueous solution of the polyvinyl alcohol-based polymer has a transparency of 80.0% or less at 20°C.
[2] A binder comprising a polyvinyl alcohol-based polymer, wherein the polyvinyl alcohol-based polymer has an average saponification value of 85.0 mol% to 89.0 mol% as measured according to JIS K6726 and meets requirement (B) : a supernatant from a fluid prepared by adding 230. 0 mL of 1-propanol to 100.0 g of a 5.0% by mass aqueous solution of the polyvinyl alcohol-based polymer has a concentration of 0.65% by mass or more at 20°C.
[3] The binder according to the above [1], wherein a supernatant from a fluid prepared by adding 230.0 mL of 1-propanol to 100.0 g of a 5.0% by mass aqueous solution of the polyvinyl alcohol-based polymer has a concentration of 0.3% by mass or more at 20°C.
[4] The binder according to the above [2], wherein a fluid prepared by adding 130.0 mL of 1-propanol to 100.0 g of a 5.0% by mass aqueous solution of the polyvinyl alcohol-based polymer has a transparency of 95.0% or less at 20°C.
[5] A binder comprising a polyvinyl alcohol-based polymer, wherein the polyvinyl alcohol-based polymer has an average saponification value of 85.0 mol% to 89.0 mol% as measured according to JIS K6726 and meets both requirement (A) : a fluid prepared by adding 130.0 mL of 1-propanol to 100.0 g of a 5.0% by mass aqueous solution of the polyvinyl alcohol-based polymer has a transparency of 80.0% or less at 20°C; and requirement (B) : a supernatant from a fluid prepared by adding 230.0 mL of 1-propanol to 100.0 g of a 5.0% by mass aqueous solution of the polyvinyl alcohol-based polymer has a concentration of 0.70% by mass or more at 20°C.
[6] The binder according to the above [1] or [5], wherein the transparency specified in requirement (A) is 50.0% or less.
[7] The binder according to the above [2] or [5], wherein the concentration of the supernatant specified in requirement (B) is 0.75% by mass or more.
[8] The binder according to any one of the above [1] to [7], wherein a 4% by mass aqueous solution of the polyvinyl alcohol-based polymer has a viscosity of 2.0 to 10.0 mPa·s as measured according to JIS K6726.
[9] The binder according to any one of the above [1] to [8], wherein the binder is used for granulation.
[10] The binder according to any one of the above [1] to [9], wherein the binder is used for granulation from powder particles having an average particle diameter of 100 µm or less.
[11] The binder according to any one of the above [1] to [10], wherein the binder is used for production of a granulated material having an average particle diameter of 300 µm or less.
[12] A method for producing a granulated material by granulation (for example, wet granulation) using the binder (or the polyvinyl alcohol-based polymer) according to any one of the above [1] to [11].
[13] The method according to the above [12], wherein the method comprises a step of bringing an aqueous and/or water-based solution containing the binder (or the polyvinyl alcohol-based polymer) into contact with powder particles.
[14] A granulated material comprising the binder (or the polyvinyl alcohol-based polymer) according to any one of the above [1] to [11] as a binder (a granulated material comprising the binder according to any one of the above [1] to [11]).
[15] The granulated material according to the above [14], wherein the binder (or the polyvinyl alcohol-based polymer) is present in an amount of 0.005 to 0.1 part by mass relative to 1 part by mass of powder particles.
[16] A tablet comprising the granulated material according to the above [14] or [15].
[17] A coated tablet comprising the tablet according to the above [16] and a polyvinyl alcohol-based polymer coating a surface of the tablet.

### ADVANTAGEOUS EFFECTS OF INVENTION

In one aspect of the present invention, a novel binder is provided. This binder comprises a PVA-based polymer but can function effectively as a binder.

In one aspect of the present invention, a binder that is useful for production of advantageous tablets is provided. For example, in the case where a granulated material (granules etc.) formed using the binder (the PVA-based polymer) of the present invention is used as a component of solid preparations (tablets etc.), advantageous solid preparations can be efficiently provided in terms of hardness and disintegrability (dissolution, moldability).

Originally, hardness and disintegrability (water disintegrability) are incompatible and have a trade-off relationship, but the binder of one aspect of the present invention can provide a good balance between hardness and disintegrability.

In one aspect of the present invention, a binder that is safe for oral and other applications is provided. The binder of the present invention comprises a PVA-based polymer having an average saponification value of 85.0 mol% to 89.0 mol%. This saponification value range of the PVA-based polymer, from 85.0 mol% to 89.0 mol%, is the same as that of commercially available PVAs for pharmaceutical use or pharmaceutical grade. This range also corresponds to the standard of the saponification value of PVA described in the official compendiums in Japan, the United States, and Europe.

### DESCRIPTION OF EMBODIMENTS

### Polyvinyl alcohol-based polymer

The binder of the present invention comprises a polyvinyl alcohol-based polymer.

The polyvinyl alcohol-based polymer (may be referred to as a PVA-based polymer, PVA, etc.) (a) may usually be a saponified product of a vinyl ester-based polymer (a polymer at least composed of a vinyl ester as a polymerizable component).

The saponification value of the PVA-based polymer is not particularly limited and is preferably within the standard of the saponification value of PVA described in the following three official compendiums: the Japan Pharmaceutical Excipient Standards, the United States Pharmacopeia, and the European Pharmacopoeia. In addition, the average saponification value of the PVA-based polymer is, for example, preferably 74.0 mol% to 89.0 mol% (e.g., 80.0 to 89.0 mol% etc.) and particularly preferably 85.0 mol% to 89.0 mol% (e.g., 85.5 to 89.0 mol%, 86.0 to 89.0 mol%, 86.5 to 89.0 mol%, 87.0 to 89.0 mol%, 87.5 to 88.9 mol%, or 88.0 to 88.8 mol%) for fast dissolution in a living body.

When the average saponification value is 85.0 mol% or more, the PVA-based polymer can be used as a material of pharmaceutical preparations adapted for global markets. In addition, the proportion of hydrophobic groups in such a PVA-based polymer is low enough so that the PVA-based polymer is highly hydrophilic, less likely to precipitate at high temperature in preparing an aqueous solution, and easy to handle . That is why the lower limit specified above is particularly preferred.

Similarly, when the average saponification value is 89.0 mol% or less, the PVA-based polymer can be used as a material of pharmaceutical preparations adapted for global markets. In addition, since the proportion of hydroxyl groups in PVA is not so high, the reduction in water solubility due to high crystallinity and the disintegrability and dissolution rate of the tablet fall into a proper range. That is why the upper limit specified above is particularly preferred.

The method for measuring the average saponification value of PVA is not particularly limited, and for example, the method for measuring the saponification value specified in JIS K6726 can be used.

In the present invention, a PVA-based polymer having a specific saponification value distribution is used.

More specifically, the PVA-based polymer meets requirement (A) and/or (B) described below. Particularly preferably, the PVA-based polymer meets at least requirement (B). The PVA-based polymer may meet both the requirements. Hereinafter, these requirements are described.

### Requirement (A)

Requirement (A): A fluid prepared by adding 130.0 mL of 1-propanol to 100.0 g of a 5.0% by mass aqueous solution of the polyvinyl alcohol-based polymer has a transparency of 80.0% or less at 20°C.

The method for preparing the aqueous solution is not particularly limited, and conventional ones may be employed. The aqueous solution may be prepared in a predetermined vessel (a beaker etc.).

The addition of (mixing with) 1-propanol can be performed in any manner with no particular limitation, and 1-propanol may be added to an aqueous solution of the polyvinyl alcohol-based polymer in a uniform state.

The addition of 1-propanol may be performed with stirring. For stirring, a stirring tool (a stir bar etc.) may be used, and a stirring means (a stirrer etc.) may be used.

The speed of the addition of 1-propanol is not particularly limited and may be, for example, 10 mL/minute.

The temperature at the time of the addition of 1-propanol (or the temperature of the fluid) is not particularly limited. The temperature may be 20°C, or at ordinary temperature or in a predetermined temperature range (e.g., 0 to 50°C, 5 to 40°C, 10 to 30°C, 15 to 25°C, etc.).

The timing of the transparency measurement is not particularly limited, and the transparency measurement may usually be performed after the fluid is allowed to stand for a predetermined time after the addition of 1-propanol. For example, after the addition of 1-propanol, stirring is stopped, the fluid is allowed to stand for a predetermined time (e.g., 10 minutes or more, such as 30 minutes), and then the transparency of the fluid is measured.

The "transparency at 20°C" may be, for example, the transparency of the fluid which has been allowed to stand at 20°C for a predetermined time (e.g., 30 minutes or more, about 30 minutes to about 1 hour) or the transparency of the fluid which has been allowed to stand at 20°C until air bubbles in the fluid are no longer visible.

A specific example is given below. 100.0 g of a 5.0% by mass aqueous polyvinyl alcohol-based polymer solution and a stirring tool [e.g., a stir bar (e.g., 4.0 cm in length)] are placed into a predetermined vessel [e.g., a 200-mL beaker (e.g., outer diameter: 6.5 cm, height: 8.8 cm)]. To this, 130.0 mL of 1-propanol is added (e.g., added dropwise) at a predetermined speed (e.g., 10 mL/minute) at ordinary temperature [e.g., 10 to 30°C (e.g., 20°C)] with stirring [e.g., using a stirrer at a predetermined rotational speed (e.g., 30 to 300 rpm, such as 100 rpm)]. After the completion of addition (e.g., at a predetermined time (e.g., 5 minutes) after the completion of addition), stirring is stopped, and the fluid is allowed to stand for a predetermined time (e.g., 30 minutes). Immediately (e.g., within not more than 10 minutes, not more than 5 minutes, not more than 3 minutes, etc.) after that, the transparency of the fluid at 20°C [e.g., the transparency of the fluid which has been allowed to stand at 20°C for a predetermined time (e.g., 30 minutes to 1 hour, such as 30 minutes)] is measured.

For the measurement of the transparency, for example, using the spectrophotometer specified in JIS K0115, the percent transmittance of light at 430 nm through the fluid in a quartz or glass absorption cell having an optical path length of 20 mm is measured and normalized to water as control.

In the case where requirement (A) is met, the transparency (the transparency at 20°C) is 80.00 or less (e.g., 75.0% or less), for example, 70.0% or less (e.g., 65% or less), preferably 60.0% or less (e.g., 55.0% or less), more preferably 50.0% or less (e.g., 45.0% or less), in particular 40.0% or less (e.g., 38.0% or less). Particularly preferably, the transparency is 35.0% or less (e.g., 33.0% or less, 32.0% or less, 31.0% or less, 30.5% or less) or 28.0% or less (e.g., 25.0% or less, 22.0% or less, 20.0% or less).

The lower limit of the transparency (the transparency at 20°C) is not particularly specified and may be 0.0%, 0.50%, 1.0%, 1.5%, 2.0%, 2.5%, 3.0%, 3.5%, 4.0%, 4.5%, 5.0%, 5.5%, or the like.

A high-saponified PVA component is hardly soluble in 1-propanol. For this reason, upon addition of a certain amount of 1-propanol to the aqueous PVA-based polymer solution, the high-saponified PVA component precipitates and produces turbidity. This means that the transparency can serve as an indicator of the amount of the high-saponified PVA component (that is, the saponification value distribution on the high value side). That is, a lower transparency indicates a larger amount of the high-saponified PVA component in the PVA-based polymer (that is, a broader tendency in saponification value distribution on the high value side).

### Requirement (B)

Requirement (B): A supernatant from a fluid prepared by adding 230.0 mL of 1-propanol to 100.0 g of a 5.0% by mass aqueous solution of the polyvinyl alcohol-based polymer has a concentration of 0.65% by mass or more (e.g., 0.70% by mass or more) at 20°C.

The method for preparing the aqueous solution is not particularly limited, and conventional ones may be employed. The aqueous solution may be prepared in a predetermined vessel (a beaker etc.).

The addition of (mixing with) 1-propanol can be performed in any manner with no particular limitation, and 1-propanol may be added to an aqueous solution of the polyvinyl alcohol-based polymer in a uniform state.

The addition of 1-propanol may be performed with stirring. For stirring, a stirring tool (a stir bar etc.) may be used, and a stirring means (a stirrer etc.) may be used.

The speed of the addition of 1-propanol is not particularly limited and may be, for example, 10 mL/minute.

The temperature at the time of the addition of 1-propanol (or the temperature of the fluid) is not particularly limited. The temperature may be 20°C, or at ordinary temperature or in a predetermined temperature range (e.g., 0 to 50°C, 5 to 40°C, 10 to 30°C, 15 to 25°C, etc.).

Regarding requirement (B), the steps before the addition of 1-propanol may be the same as those described above for requirement (A).

The supernatant to be subjected to concentration measurement can be obtained by, for example, allowing the fluid (mixture) to stand for a predetermined time [e.g., a sufficient time such as 3 hours or more (e.g., 24 hours)] after the addition of 1-propanol. In order to obtain the supernatant efficiently (certainly), for example, centrifugation is preferably employed. Centrifugation of the mixture provides the supernatant efficiently (and certainly).

The conditions of the centrifugation are not particularly limited as long as the supernatant can be obtained by the centrifugation. For example, the centrifugation (centrifugal treatment) may be performed at a predetermined rotational speed (e.g., 1000 rpm or more, 3000 rpm or more, 5000 rpm or more, 10000 rpm or more, such as 14000 rpm, etc.) for a predetermined time (e.g., 1 minute or more, 3 minutes or more, 5 minutes or more, 7 minutes or more, 10 minutes or more, such as 10 minutes, etc.).

The centrifuge (centrifugal machine) is not particularly limited, and for example, "H-9R" manufactured by KOKUSAN Co. Ltd. may be used.

The "supernatant at 20°C" may be, for example, the supernatant of the fluid which has been allowed to stand at 20°C for a predetermined time (e.g., 30 minutes or more, about 30 minutes to about 1 hour) or the supernatant of the fluid which has been allowed to stand at 20°C until air bubbles in the fluid are no longer visible. As described above, centrifugation may be further performed at 20°C on the mixture which has been allowed to stand.

A specific example is given below. 100.0 g of a 5.0% by mass aqueous polyvinyl alcohol-based polymer solution and a stirring tool [e.g., a stir bar (e.g., 4.0 cm in length)] are placed into a predetermined vessel [e.g., a 200-mL beaker (e.g., inner diameter: 6.5 cm, height: 8.8 cm)]. To this, 130.0 mL of 1-propanol is added (e.g., added dropwise) at a predetermined speed (e.g., 10 mL/minute) at ordinary temperature [e.g., 10 to 30°C (e.g., 20°C)] with stirring [e.g., using a stirrer at a predetermined rotational speed (e.g., 30 to 300 rpm, such as 100 rpm)]. After the completion of addition (e.g., at a predetermined time (e.g., 5 minutes) after the completion of addition), stirring is stopped, and the fluid is allowed to stand at 20°C for a predetermined time (e.g., 30 minutes). After that, the supernatant {e.g., the supernatant obtained after centrifugation [e.g., centrifugation performed using a centrifugal machine (e.g., "H-9R" manufactured by KOKUSAN Co. Ltd.) at 20°C at a predetermined rotational speed (e.g., 14000 rpm) for a predetermined time (e.g., 10 minutes)] is subjected to the concentration measurement.

The supernatant used for the concentration measurement may be the whole supernatant separated from the mixture (separated from the precipitate in the mixture) or a portion (part) of the supernatant separated from the mixture.

The method for measuring the concentration (solid content) of the supernatant is not particularly limited. For example, from a predetermined amount of the supernatant (the separated supernatant), the solvent components (water and 1-propanol) are removed, and the concentration is determined (calculated) from the mass of the supernatant measured before and after the removal of the solvent components.

An example of the method for measuring the concentration of the supernatant is shown below.

A predetermined amount (e.g., about 80 g) of the supernatant is taken using a Pasteur pipette with caution not to take the precipitate (and gently placed on a dish, for example), followed by drying or solvent removal (e.g., drying at 60°C for 5 hours and then at 105°C for 24 hours). From the mass of the sample supernatant and the change in mass before and after drying (solvent removal), the concentration is calculated.

In the case where requirement (B) is met, the concentration of the supernatant (the concentration of the supernatant at 20°C) is 0.65% by mass or more (e.g., 0.68% by mass or more), 0.70% by mass or more (e.g., 0.71% by mass or more), or the like. For example, the concentration of the supernatant is 0.72% by mass or more (e.g., 0.73% by mass or more), preferably 0.74% by mass or more (e.g., 0.75% by mass or more), more preferably 0.76% by mass or more (e.g., 0.77% by mass or more), in particular 0.78% by mass or more (e.g., 0.79% by mass or more). Particularly preferably, the concentration of the supernatant is 0.80% by mass or more (e.g., 0.81% by mass or more) or 0.82% by mass or more (e.g., 0.83% by mass or more, 0.84% by mass or more).

The upper limit of the concentration of the supernatant (the concentration of the supernatant at 20°C) is not particularly specified and may be 2.0% by mass, 1.8% by mass, 1.6% by mass, 1.5% by mass, 1.3% by mass, 1.2% by mass, 1.1% by mass, 1.0% by mass, 0.95% by mass, 0.90% by mass, 0.88% by mass, 0.87% by mass, 0.86% by mass, 0.85% by mass, or the like.

A low-saponified PVA component hardly precipitates even after 1-propanol is added. For this reason, upon addition of a certain amount of 1-propanol to the aqueous PVA-based polymer solution, the low-saponified PVA component is still present in a dissolved state in the fluid. This means that the concentration of the supernatant can serve as an indicator of the amount of the low-saponified PVA component (that is, the saponification value distribution on the low value side) . That is, a higher concentration of the supernatant indicates a larger amount of the low-saponified PVA component in the PVA-based polymer (that is, a broader tendency in saponification value distribution on the low value side).

Thus, the PVA-based polymer that meets requirement (A) and/or (B) can be considered (presumed) to have a broader saponification value distribution. In particular, the PVA-based polymer that meets at least requirement (B) (that is, has a broader saponification value distribution on the low value side) has a higher potential for efficiently providing or exerting binder functions.

Given this perspective, at least one of requirements (A) and (B) has to be met, but both of them do not have to be met. That is, in the case where the PVA-based polymer meets requirement (A) (or requirement (B)), it is optional whether or not the PVA-based polymer meets requirement (B) (or requirement (A)).

In the case where both requirements are met, the PVA-based polymer may have a higher potential for providing or exerting binder functions at an even more higher level, but as long as either one of the requirements (e.g., requirement (B)) is met, the PVA-based polymer can provide or exert binder functions.

From this viewpoint, in order to efficiently provide or exert binder functions, even in the case where both requirements are not met, it is preferable that the PVA-based polymer is slightly short of the unmet requirement (does not deviate largely from the unmet requirement).

For example, in the case where the PVA-based polymer meets requirement (B), it is optional whether or not the PVA-based polymer meets requirement (A) [that is, the transparency (the transparency at 20°C) may be more than 80.0%], but even in the case where requirement (A) is unmet, it is preferable that the transparency (the transparency at 20°C) is not too high (e.g., 98.0% or less, 97.0% or less, 96.0% or less, 95.0% or less, 93.0% or less, 90.0% or less, 88.0% or less, 87.0% or less, 86.0% or less, 85.0% or less, 84.0% or less, 83.0% or less, 82.0% or less, 81.0% or less, or the like).

In the case where the PVA-based polymer meets requirement (A), it is optional whether or not the PVA-based polymer meets requirement (B) [that is, the concentration of the supernatant (the concentration of the supernatant at 20°C) may be less than 0.65% by mass], but even in the case where requirement (B) is unmet, it is preferable that the concentration of the supernatant (the concentration of the supernatant at 20°C) is not too low (e.g., 0.1% by mass or more, 0.2% by mass or more, 0.25% by mass or more, 0.3% by mass or more, 0.35% by mass or more, 0.4% by mass or more, 0.45% by mass or more, 0.5% by mass or more, 0.55% by mass or more, 0.6% by mass or more, 0.61% by mass or more, 0.62% by mass or more, 0.63% by mass or more, 0.64% by mass or more, 0.65% by mass or more, 0.66% by mass or more, 0.67% by mass or more, 0.68% by mass or more, 0.69% by mass or more, or the like).

The viscosity of a 4% by mass aqueous solution of the PVA-based polymer (as measured according to JIS K6726) is not particularly limited. The viscosity is preferably 2.0 to 10.0 mPa·s (e.g., 2.2 to 9.5 mPa·s, 2.5 to 9.0 mPa·s, or 2.8 to 8.5 mPa·s) and may be 3.0 to 7.0 mPa·s or less (e.g., 3.5 to 6.5 mPa·s, 4.0 to 6.0 mPa·s, or 4.5 to 5.5 mPa·s) .

When the 4% by mass aqueous solution has such a viscosity and the above-described requirement (A) and/or (B) is met, shorter disintegration time of tablets is efficiently and easily achieved.

The PVA-based polymer used may be obtained commercially or synthesized. The method for producing the PVA-based polymer is not particularly limited, and known methods, for example, saponification of a polymer containing a vinyl ester monomer as a polymerizable component (vinyl ester-based polymer), may be used.

The vinyl ester monomer is not particularly limited, and examples include fatty acid vinyl esters [e.g., C₁₋₂₀ fatty acid vinyl esters (e.g., C₁₋₁₆ alkanoic acid vinyl esters) such as vinyl formate, vinyl acetate, vinyl propionate, vinyl butyrate, vinyl caprylate, vinyl versatate, and vinyl monochloroacetate], and aromatic carboxylic acid vinyl esters [e.g., vinyl arenecarboxylates (e.g., C₇₋₁₂ arene carboxylic acid vinyl esters) such as vinyl benzoate].

One kind of vinyl ester monomer or a combination of two or more kinds of vinyl ester monomers may be used.

The vinyl ester monomer preferably at least contains a fatty acid vinyl ester (e.g., C₁₋₁₀ alkanoic acid vinyl esters etc., such as vinyl formate, vinyl acetate, vinyl propionate, and vinyl butyrate). Particularly, vinyl acetate is preferred from industrial or other viewpoints.

The vinyl ester-based polymer has a vinyl ester unit, and if necessary, may have an additional monomer unit (a monomer capable of copolymerizing with vinyl ester monomers) (in other words, the vinyl ester-based polymer may be modified with an additional monomer).

The additional monomer is not particularly limited, and examples include, but are not limited to, α-olefins (e.g., ethylene, propylene, etc.), (meth)acrylic acid esters [e.g., (meth) acrylic acid alkyl esters such as methyl (meth) acrylate, ethyl (meth)acrylate, butyl (meth)acrylate, and 2-ethylhexyl (meth)acrylate], unsaturated amides [e.g., (meth)acrylamide, diacetone acrylamide, N-methylolacrylamide, etc.], unsaturated acids {e.g., unsaturated acids [e.g., (meth) acrylic acid, crotonic acid, maleic acid, itaconic acid, fumaric acid, etc.], unsaturated acid esters [unsaturated acid esters other than (meth)acrylic acid esters, e.g., alkyl (methyl, ethyl, propyl, etc.) esters etc.], unsaturated acid anhydrides (maleic anhydride etc.), salts of unsaturated acids [e.g., alkali metal salts (e.g., sodium salts, potassium salts, etc.), ammonium salts, etc.], etc.}, glycidyl group-containing monomers [e.g., allyl glycidyl ethers, glycidyl (meth)acrylate, etc.], sulfonic group-containing monomers (e.g., 2-acrylamide-2-methylpropane sulfonic acid, salts thereof, etc.), phosphate group-containing monomers [e.g., acid phosphoxy ethyl (meth)acrylate, acid phosphoxy propyl (meth) acrylate, etc.], vinyl ethers (e.g., alkyl vinyl ethers), allyl alcohols, etc.

One kind of additional monomer or a combination of two or more kinds of additional monomers may be used.

In the case where the additional monomer is contained as a polymerizable component in the vinyl ester-based polymer, the proportion of the additional monomer in the polymerizable components may be, for example, 50% by mass or less, 30% by mass or less, 20% by mass or less, or 10% by mass or less.

The proportion of the vinyl ester monomer in the polymerizable components is, for example, 50% by mass or more, preferably 70% by mass or more, more preferably 90% by mass or more and may be 100% by mass.

In the PVA-based polymer, some vinyl alcohol units may be modified by a reaction, such as acetalization, etherification, acetoacetylization, or cationization.

The method for polymerizing the polymerizable components (e.g., the polymerizable components including the vinyl ester monomer such as vinyl acetate) is not particularly limited, and examples include known polymerization methods such as block polymerization, solution polymerization, suspension polymerization, and emulsion polymerization. Among them, solution polymerization using methanol as a solvent is industrially preferred. In the solution polymerization, known initiators such as peroxide initiators and azo initiators can be used, and the polymerization degree of the vinyl ester-based polymer can be adjusted by varying the feed ratio of the polymerizable components and methanol and the polymerization yield. For the production of the PVA-based polymer, commercial vinyl ester-based polymers (polyvinyl acetate resin etc.) can be used as a starting material.

The method for saponifying the vinyl ester-based polymer (e.g., polyvinyl acetate) can be a conventional saponification method using an alkaline or acid catalyst. In particular, industrially preferred is alcoholysis, which is performed by adding an alkali such as sodium hydroxide to a solution of the vinyl ester-based polymer (e.g., polyvinyl acetate) in methanol or in a mixed solvent of methanol, water, methyl acetate, etc. and stirring the mixture for cleavage of the acyl group (e.g., acetyl group) of the vinyl ester-based polymer (e.g., polyvinyl acetate).

After that, the obtained mass product, gelled product, or granular product is pulverized, and if needed, the alkali is neutralized; then the solid is separated from the liquid and dried to yield a PVA-based polymer.

The PVA-based polymer used in the present invention can efficiently be produced by performing saponification in a less homogenous system than usual. For such saponification, the following approaches can be employed: saponification is performed using a methanol solution of the vinyl ester-based polymer (e.g., polyvinyl acetate) at a higher concentration (e.g., 55% by mass or more); saponification is performed with stirring at a lower speed (e.g., 20 rpm or less) after addition of an alkali; saponification is performed for a shorter time for mixing and stirring after the addition of an alkali; saponification is performed using a larger amount of an alkali for a shorter time; and saponification is performed in a reaction system having a temperature gradient or distribution created by, for example, adjusting the temperatures of a methanol solution of the vinyl ester-based polymer (e.g., polyvinyl acetate) and an alkali to be added to the solution.

Other approaches are available, and for example, saponification may be performed under the conditions that the solvents added, such as water and methyl acetate, are in a less homogenous state, in view of the fact that the homogeneity of the solvents affects the saponification speed. These approaches easily achieve the variation in saponification value in the PVA-based polymer after saponification. This means that the PVA-based polymer produced by any of these approaches has the same average saponification value as that of the PVA-based polymer produced by the conventional method but a broader saponification value distribution.

In addition to the above-described approaches, for a broader saponification value distribution, two or more types of PVAs having different saponification values may be blended such that the weighted average saponification value falls into the desired value. This can be used as one embodiment of the PVA-based polymer in the present invention.

In this case, the PVA-based polymer can be obtained by mixing a plurality of types of PVAs, for example, two different PVAs, PVA (a) and PVA (b).

The average saponification value of the PVA (a) as measured according to the method for measuring the saponification value specified in JIS K6726 is, for example, 85 mol% or more (e.g., 85 to 99 mol%), preferably 88 mol% or more (e.g., 88 to 99 mol%), more preferably 90 mol% or more (e.g., 90 to 99 mol%), and still more preferably 92 mol% or more (e.g., 92 to 99 mol%).

The average saponification value of the PVA (b) as measured in the same manner as above is, for example, 99 mol% or less (e.g., 60 to 99 mol%), preferably 95 mol% or less (e.g., 60 to 95 mol%), more preferably 90 mol% or less (e.g., 65 to 90 mol%), and still more preferably 88 mol% or less (e.g., 65 to 88 mol%) .

The ratio of PVA (a) and PVA (b) can be selected depending on whether requirement (A) or (B) or both are met. The mass ratio of PVA (a) :PVA(b) is, for example, 5:95 to 95:5, preferably 10:90 to 90:10, more preferably 15:85 to 85:15, and still more preferably 20:80 to 80:20.

The weighted average saponification value of the PVA-based polymer obtained by mixing PVA (a) and PVA (b) (when the saponification value of PVA (a) is A mol%, the saponification value of PVA (b) is B mol%, and the ratio of PVA (a) and PVA (b) is A':B', the weighted average saponification value C is expressed as C = (A × A' + B × B')/100) can be selected from the same range as described above and is, for example, 83.0 to 89.0 mol%, preferably 85.0 to 89.0 mol%, more preferably 86.0 to 89.0 mol%.

### Binder etc.

The binder of the present invention comprises the PVA-based polymer.

As long as the binder of the present invention functions as a binder for a substance to be mixed therewith (e.g., a powder), the application of the binder is not particularly limited, but the binder is particularly suitable for use as a binder for granulation.

The substance to be mixed with the binder is not particularly limited and may be, for example, a pharmaceutical product, a quasi-pharmaceutical product, a food product, or the like. The substance to be mixed with the binder may be an active ingredient, a nutrient (or nutritional ingredient), or the like, and may be a medicinal substance (an active pharmaceutical ingredient etc.).

The substance to be mixed with the binder may be an organic substance or an inorganic substance, a mixture thereof, or an organic-inorganic hybrid material.

The medicinal substance or the active ingredient (or the medicinal substance or the active ingredient contained in the substance to be mixed with the binder) is not particularly limited. Examples of the medicinal substance include central nervous system drugs, cardiovascular drugs, respiratory drugs, gastrointestinal drugs, antibiotics, antitussives and expectorants, antihistamines, antipyretic, analgesic, and antiphlogistic drugs, diuretics, autonomic drugs, antimalarials, antidiarrheals, psychotropics, and vitamins and derivatives thereof.

Examples of the central nervous system drug include diazepam, idebenone, aspirin, ibuprofen, paracetamol, naproxen, piroxicam, indomethacin, sulindac, lorazepam, nitrazepam, phenytoin, acetaminophen, ethenzamide, ketoprofen, and chlordiazepoxide.

Examples of the cardiovascular drug include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, propranolol hydrochloride, and alprenolol hydrochloride.

Examples of the respiratory drug include amlexanox, dextromethorphan, theophylline, pseudoephedrine, salbutamol, and guaifenesin.

Examples of the gastrointestinal drug include benzimidazole drugs with antiulcer activity, such as 2-[[3-methyl-4-(2,2,2-trifluoroethoxy)-2-pyridyl]methylsulf inyl]benzimidazole and 5-methoxy-2-[(4-methoxy-3,5-dimethyl-2-pyridyl)methylsulfin yl]benzimidazole, cimetidine, ranitidine, pirenzepine hydrochloride, pancreatin, bisacodyl, and 5-aminosalicylic acid.

Examples of the antibiotic include talampicillin hydrochloride, bacampicillin hydrochloride, cefaclor, and erythromycin.

Examples of the antitussive and expectorant include noscapine hydrochloride, carbetapentane citrate, dextromethorphan hydrobromide, isoaminile citrate, and dimemorfan phosphate.

Examples of the antihistamine include chlorpheniramine maleate, diphenhydramine hydrochloride, and promethazine hydrochloride.

Examples of the antipyretic, analgesic and antiphlogistic drug include ibuprofen, diclofenac sodium, flufenamic acid, sulpyrine, aspirin, and ketoprofen.

Examples of the diuretic include caffeine.

Examples of the autonomic drug include dihydrocodeine phosphate, dl-methylephedrine hydrochloride, atropine sulfate, acetylcholine chloride, and neostigmine.

Examples of the antimalarial include quinine hydrochloride.

Examples of the antidiarrheal include loperamide hydrochloride.

Examples of the psychotropic include chlorpromazine.

Examples of the vitamin and a derivative thereof include vitamin A, vitamin B1, fursultiamine, vitamin B2, vitamin B6, vitamin B12, vitamin C, vitamin D, vitamin E, vitamin K, calcium pantothenate, and tranexamic acid.

In the case where the substance to be mixed with the binder contains an active ingredient, a nutrient, etc., the substance to be mixed with the binder may further contain an additional ingredient (any additive usually used in this field, for example, a filler, a disintegrant, a lubricant, an antiagglomerant, a solubilizer, etc.).

Examples of the filler include saccharides, such as sucrose, lactose, mannitol, and glucose, starch, crystalline cellulose, calcium phosphate, calcium sulfate, etc. Examples of the disintegrant include low-substituted hydroxypropyl cellulose, carmellose or a salt thereof, croscarmellose sodium, sodium carboxymethyl starch, crospovidone, microcrystalline cellulose, microcrystalline cellulose-carmellose sodium, etc. Examples of the lubricant or the antiagglomerant include talc, magnesium stearate, calcium stearate, colloidal silica, stearic acid, waxes, hydrogenated oils, polyethylene glycols, sodium benzoate, etc. Examples of the solubilizer include organic acids, such as fumaric acid, succinic acid, malic acid, and adipic acid, etc. One of these additives or two or more of them may be used. The amount of the additional ingredient (the additive) can be determined as appropriate according to, for example, the type of the drug.

One kind of substance to be mixed with the binder or a combination of two or more kinds of substances to be mixed with the binder may be used.

The substance to be mixed with the binder may be a solid at ordinary temperature (e.g., 10 to 40°C etc.).

The substance to be mixed with the binder may be, for example, in the form of powder (or powder particles) . The particle size of the powder (active ingredient, active ingredient powder, etc.) is not particularly limited, and the average particle diameter is, for example, about 500 µm or less (e.g., 5 to 400 µm), preferably about 300 µm or less, more preferably about 100 µm or less (e.g., 10 to 80 µm).

The average particle diameter of the powder may be measured with a laser-type particle size distribution measuring instrument etc.

In the case where the binder is used for granulation, granulated materials (granular powder) can be obtained. That is, the granulated material comprises a substance to be mixed with the binder (powder particles etc.) and the binder. In other words, the granulated material can also be called a granulated material in which the binder is the binder of the present invention (or the polyvinyl alcohol-based polymer).

A powdery granulated material (granular powder) may usually be in the form of a granule (granular shape).

The particle size of the granulated material (e.g., granules) can be determined as appropriate according to the size of the powder particles, the granulation method, etc. The average particle diameter is, for example, about 700 µm or less (e.g., 30 to 600 µm), preferably about 400 µm or less, and more preferably about 300 µm or less (e.g., 50 to 200 µm).

The average particle diameter of the granulated material may be measured with a laser-type particle size distribution measuring instrument etc.

The proportion of the binder (or the polyvinyl alcohol-based polymer) in the granulated material can be selected from the range of, for example, about 0.1 to 50% by mass, may be about 0.2 to 30% by mass (e.g., 0.3 to 15% by mass), and is preferably about 0.5 to 10% by mass (e.g., 0.7 to 8% by mass), more preferably about 1 to 5% by mass (e.g., 1 to 3% by mass).

The proportion of the binder (or the polyvinyl alcohol-based polymer) of the present invention in the granulated material can be selected from the range of, for example, about 0.001 to 0.5 part by mass relative to 1 part by mass of the powder particles, may be about 0.002 to 0.2 part by mass (e.g., 0.003 to 0.15 part by mass), and is preferably about 0.005 to 0.1 part by mass (e.g., 0.007 to 0.08 part by mass), more preferably about 0.01 to 0.05 part by mass (e.g., 0.01 to 0.03 part by mass).

The binder of the present invention is not particularly limited in usage as long as it functions as a binder for the substance to be mixed therewith. Usually, the binder of the present invention is used by contact with the substance to be mixed therewith.

In particular, by using the binder (or the polyvinyl alcohol-based polymer) of the present invention for granulation (bringing the binder into contact with the substance to be mixed therewith), a granulated material can be produced.

The use of the binder (the granulated material) of the present invention optionally includes the following embodiments (1) to (3):
(1) use for levodopa and/or carbidopa (e.g., incorporation a polymer mixture composed of polyvinyl alcohols having different saponification values into a levodopa or carbidopa-containing pharmaceutical preparation);
(2) use for irbesartan (e.g., incorporation into an irbesartan-containing pharmaceutical composition); and
(3) use for obeticholic acid or a pharmaceutically acceptable salt thereof (e.g., use as a water-soluble polymer binder (corresponding to (iii) below) in an oral pharmaceutical preparation containing obeticholic acid or a pharmaceutically acceptable salt thereof, (i) a water-soluble filler, (ii) a disintegrant, and (iii) a water-soluble polymer binder.

The granulation method is not particularly limited, and dry granulation (e.g., powder (such as medicinal substance powder) and a binder are mixed, the powder mixture is compacted by applying a force with an apparatus called a roller compactor to form larger particles, and the particles are ground to produce granules) may be used. Typically, wet granulation may be used.

Wet granulation at least includes a step of bringing the binder in a liquid form into contact with the substance to be mixed therewith (granulation step). In a typical granulation step, the binder (or the polyvinyl alcohol-based polymer) may be brought into contact with the substance to be mixed therewith in the presence of a solvent. More specifically, a dispersion or solution (in particular, an aqueous and/or water-based solution) containing the binder may be brought into contact with (e.g., sprayed onto or added dropwise onto) the substance to be mixed therewith (in particular, powder particles) (e.g., solution addition method). Alternatively, a solvent (a solvent which can dissolve or disperse the binder) may be brought into contact with (e.g., sprayed onto or added dropwise onto) a mixture of the binder and the substance to be mixed therewith (powder particles) (e.g., powder addition method).

The solvent is not particularly limited as long as it can dissolve or disperse the binder (or the polyvinyl alcohol-based polymer). Usually, water or a mixed solvent (water-based solvent) of water and an organic solvent is suitable, and in particular, water or a water-based solvent (typically water) is preferably suitable. The organic solvent (water-miscible solvent) is not particularly limited, and examples include alcohols (e.g., ethanol etc.) etc.

In the wet granulation, the proportion or the amount of the solvent used (e.g., the proportion of water in an aqueous solution) is, for example, about 1 to 70 parts by mass (e.g., 3 to 60 parts by mass), preferably about 4 to 50 parts by mass (e.g., 5 to 45 parts by mass), and more preferably about 7 to 40 parts by mass (e.g., 8 to 35 parts by mass) relative to 100 parts by mass of the substance to be mixed with the binder (powder particles).

Specific examples of the wet granulation include fluid bed granulation, high-speed stirring granulation, extrusion granulation, tumbling granulation, etc. In particular, fluid bed granulation, high-speed stirring granulation, etc. are suitable in the present invention.

Fluid bed granulation is performed, for example, in the following procedure: the substance to be mixed with the binder (powder) is fed into a layer called a fluid bed; and while warm air flow is supplied from the bottom to fluidize the substance to be mixed with the binder (powder), a solution or dispersion (in particular, an aqueous solution) containing the binder is sprayed from the above to form granules. In fluid bed granulation, drying is performed concomitantly with granulation (a step of forming granules), and the time of drying can be saved after granulation.

For high-speed stirring granulation, a high-speed stirring granulator with a large blade called a blender and a small blade called a chopper inside of the apparatus is used, for example. In such high-speed stirring granulation, the substance to be mixed with the binder (powder) is fed into the apparatus, and while the stirring blades are rotated, a solution or dispersion (in particular, aqueous solution) containing the binder is sprayed or added dropwise to form granules. After granulation, drying is performed and thus finished granules are obtained.

The granulated material (e.g., granules) can be used in various ways according to the type of the substance to be mixed with the binder, the desired application, etc. For example, the granulated material may be directly used as granules (e.g., granules, granules in a capsule, etc.) or molded into any form.

For example, the granulated material (granules) can be used as a material for tablets. In other words, such a tablet contains the granulated material and can be produced from the granulated material by tableting.

The granulated material (and a molded product therefrom such as a tablet) of the present invention is suitable for oral application (oral preparations).

The tablet contains at least the granulated material and optionally an additional ingredient.

The additional ingredient may be any additive usually used in this field (e.g., a disintegrant, a lubricant, an antiagglomerant, a solubilizer, etc.).

Examples of the disintegrant include low-substituted hydroxypropyl cellulose, carmellose or a salt thereof, croscarmellose sodium, sodium carboxymethyl starch, crospovidone, microcrystalline cellulose, microcrystalline cellulose-carmellose sodium, etc.

Examples of the lubricant or the antiagglomerant include talc, magnesium stearate, calcium stearate, colloidal silica, stearic acid, waxes, hydrogenated oils, polyethylene glycols, sodium benzoate, etc.

Examples of the solubilizer include organic acids, such as fumaric acid, succinic acid, malic acid, and adipic acid, etc.

One of these additives or two or more of them may be used.

The amount of the additive can be determined as appropriate according to, for example, the kind(s) of the ingredient(s) contained in the tablet or in the granulated material.

The proportion of the binder (or the polyvinyl alcohol-based polymer) in the tablet is, for example, about 0.5 to 10% by mass, preferably about 2 to 6% by mass (e.g., 1 to 5% by mass), more preferably about 1 to 3% by mass and may be 3% by mass or less (e.g., 1 to 2% by mass, 2 to 3% by mass, etc.).

The shape of the tablet is not particularly limited and may be any shape, such as a disk shape, a lens shape, or a pole shape.

The size of the tablet is also not particularly limited and may be, for example, 3 mm or more (e.g., 4 to 15 mm, 5 to 12 mm, 8 to 11 mm, etc.) in diameter (maximum diameter).

The tableting method is not particularly limited, and conventional tableting methods can be employed.

The tablet may have a coat layer on the surface of the tablet. Therefore, the present invention further includes a coated tablet comprising a tablet and a coat layer (coating layer) coating a surface of the tablet.

The component of the coat layer in the coated tablet is not particularly limited, and examples include resin materials, such as cellulose resin (e.g., hydroxypropylmethyl cellulose, hydroxypropylmethylcellulose acetate succinate etc.), and polyvinyl alcohol-based polymers.

In the case where the coat layer comprises a polyvinyl alcohol-based polymer, the polyvinyl alcohol-based polymer optionally but preferably meets the above-described requirement (A) and/or (B). In the case where the coat layer is composed of a polyvinyl alcohol-based polymer which meets requirement (A) and/or (B), the coat layer can be formed in an efficient and highly productive manner, prevent adhesion between tablets, and provide moisture-proofing and gas barrier properties.

The method for forming the coat layer on the surface of the tablet (the method for producing the coated tablet) is not particularly limited, and known types of coating, for example, film coating etc. may be used.

The coating technique may be, for example, spray coating.

The coating apparatus used may be, for example, a pan coater, a drum coater, or the like. These apparatuses may be equipped with an air spray, an airless spray, or other types of spray devices.

A specific example of the method for forming the coat layer is given below. A coating composition which contains the components of the coat layer, and if necessary, an additive, is dissolved or dispersed in a solvent [e.g., water, an organic solvent (e.g., an alcohol such as ethanol etc.), a mixed solvent of water and an organic solvent, etc.] to prepare a coating composition solution, and the solution is sprayed or applied to a tablet using the above-mentioned coating apparatus in parallel with drying to coat the surface of the tablet.

The coating weight of the coating composition for coating the surface of the tablet varies with the type, form, size, and surface condition of the tablet, the properties of the components and additives contained in the tablet, and other factors. For example, the coating weight is preferably 1 to 10% by mass, more preferably 1 to 7% by mass, and particularly preferably 2 to 6% by mass relative to the total weight of the tablet. When the coating weight is within this range, perfect coating is achieved, and sufficient moisture-proofing, oxygen barrier, and odor masking are provided. In addition, the time for coating is shortened. That is why the range specified above is preferred.

The coated tablet may have a multi-layered structure having the coat layer and an additional layer. The additional layer may be, for example, an undercoat layer (a layer formed on the lower surface of the coat layer) and/or an overcoat layer (a layer formed on the upper surface of the coat layer). The additional layer can also be composed of the same components as those of the coat layer.

### EXAMPLES

Hereinafter, the present invention will be described in more detail by examples, but the present invention is not limited thereto.

In the following Examples and Comparative Examples, "percentage (%)" and "part" are on a mass basis unless otherwise specified.

The experimental conditions are as follows.

### Average saponification value and 4% by mass aqueous solution viscosity

The measurements were performed according to JIS K6726.

### Transparency (confirmation of whether or not requirement (A) is met)

Into a 200-mL beaker (inner diameter: 6.5 cm, height: 8.8cm), 100.0 g of a 5.0% by mass aqueous PVA-based polymer solution was placed, and a 4. 0-cm stir bar was added. While the solution was stirred with a stirrer at 100 rpm to keep the aqueous solution in a uniform state, 130.0 mL of 1-propanol was added dropwise at a speed of 10 mL/minute at ordinary temperature (about 20°C) . Stirring was stopped 5 minutes after the completion of dropwise addition, and the fluid was allowed to stand for 30 minutes and continuously allowed to stand at 20°C for 30 minutes. Immediately (not more than 1 minute) after that, the transparency of the fluid at 20°C was measured according to the following method.

### Transparency:

Using the spectrophotometer specified in JIS K0115, the percent transmittance of light at 430 nm through the fluid in a glass absorption cell having an optical path length of 20 mm was measured and normalized to water as control.

### Concentration of supernatant (confirmation of whether or not requirement (B) is met)

Into a 200-mL beaker (inner diameter: 6.5 cm, height: 8.8cm), 100.0 g of a 5.0% by mass aqueous PVA-based polymer solution was placed, and a 4. 0-cm stir bar was added. While the solution was stirred with a stirrer at 100 rpm to keep the aqueous solution in a uniform state, 130.0 mL of 1-propanol was added dropwise at a speed of 10 mL/minute at ordinary temperature (about 20°C) . Stirring was stopped 5 minutes after the completion of dropwise addition, and the fluid was allowed to stand at 20°C for 30 minutes. The fluid was centrifuged using "H-9R" manufactured by KOKUSAN Co. Ltd. at 14000 rpm for 10 minutes to separate the supernatant from the precipitate. The concentration of the supernatant was measured according to the following method.

### Concentration of supernatant:

The supernatant (about 80 g) was taken using a Pasteur pipette with caution not to take the precipitate and gently placed on a dish. The sample supernatant was dried at 60°C for 5 hours and then at 105°C for 24 hours. From the mass of the sample supernatant and the change in mass before and after drying, the concentration was calculated.

### Granulation conditions

Granulator: MP-01 (Powrex)
Inlet air temperature: 55 to 64°C
Outlet air temperature: 28 to 34°C
Inlet air flow: 0.8 to 1.0 m³/min
Spray air flow: 35 L/min
Spray air pressure: 0.11 MPa
Amount of aqueous binder solution: 250 g
Concentration of aqueous binder solution: 4 wt%
Drying conditions after granulation: after the completion of granulation, drying is performed until the outlet temperature reaches 40°C.

### Tableting conditions

Tablet press: VERGO (manufactured by KIKUSUI SEISAKUSHO LTD.)
Tableting pressure: 10 kN
Tablet press rotational speed: 10 rpm
Tablet weight: 200 mg

### Evaluation of tablet hardness

The hardness of the obtained tablet was measured using a tablet hardness tester (TBH125 manufactured by ERWEKA). The measurement was performed 6 times and the average value of the 6 results was used as the measured value.

### Evaluation of disintegration time

The disintegration time of the obtained tablet was measured using a disintegration tester (NT400 manufactured by Toyama Sangyo Co., Ltd.). For the measurement, the disintegration test method of the Japanese Pharmacopoeia was used as a test method, and pure water (purified water) was used as a test medium. The measurement was performed 6 times and the average value of the 6 results was used as the measured value.

### Synthesis methods of PVA-based polymers

### Comparative Synthesis Example 1

A commercial polyvinyl acetate resin (manufactured by JAPAN VAM & POVAL CO., LTD., JMR-30LL, polymerization degree: 590) was dried in vacuo at 100°C for water removal. This was dissolved in methanol to prepare a 46% by mass methanol solution of polyvinyl acetate. For saponification, 500 parts by mass of this solution was heated to 40°C, and 16 parts by mass of a 3% by mass methanol solution of sodium hydroxide adjusted to 35°C was added. The mixture was stirred using a propeller-type blade at 300 rpm for 1 minute and then allowed to stand at 40°C for 40 minutes. The obtained gelled material was ground and soaked in a mixed solvent composed of 570 parts by mass of methanol, 230 parts by mass of methyl acetate, and 17 parts by mass of water. With gentle stirring, saponification was further performed at 40°C for 1 hour. The reaction mixture was neutralized to a pH of 8 to 9 with a 1% by mass aqueous acetic acid solution. The solid was separated from the liquid and dried at 60°C for 8 hours to give a PVA-based polymer.

The measured physical properties of the obtained PVA-based polymer are shown in Table 1.

### Synthesis Example 1

A PVA-based polymer was obtained in the same manner as described in Comparative Synthesis Example 1 except that the mixture of the methanol solution of polyvinyl acetate and the sodium hydroxide solution was stirred at a speed of 60 rpm for 30 seconds, that is, stirring was performed under the conditions to keep the mixture less homogeneous than usual.

The measured physical properties of the obtained PVA-based polymer are shown in Table 1.

### Synthesis Example 2

A PVA-based polymer was obtained in the same manner as described in Comparative Synthesis Example 1 except that the mixture of the methanol solution of polyvinyl acetate and the sodium hydroxide solution was stirred at a speed of 20 rpm for 60 seconds, that is, stirring was performed under the conditions to keep the mixture less homogeneous than usual.

The measured physical properties of the obtained PVA-based polymer are shown in Table 1.

### Synthesis Example 3

A PVA-based polymer was obtained in the same manner as described in Comparative Synthesis Example 1 except that saponification was performed using a mixture of 500 parts by mass of a 55% by mass methanol solution of polyvinyl acetate and 23 parts by mass of the sodium hydroxide solution.

The measured physical properties of the obtained PVA-based polymer are shown in Table 1.

### Synthesis Example 4

A PVA-based polymer was obtained in the same manner as described in Comparative Synthesis Example 1 except that saponification was performed with the following changes : a band heater was wrapped around the upper half of the vessel containing the mixture of the methanol solution of polyvinyl acetate and the sodium hydroxide solution to heat the upper half to 50°C, so that a temperature gradient in which the temperature of the upper half was 50°C and the temperature of the lower half was room temperature (25°C) was applied during the reaction; and the mixture was allowed to stand for 50 minutes.

The measured physical properties of the obtained PVA-based polymer are shown in Table 1.

### Synthesis Example 5

A PVA-based polymer was obtained in the same manner as described in Comparative Synthesis Example 1 except for the following changes : 500 parts by mass of the 46% by mass methanol solution of polyvinyl acetate was equally divided into two portions (250 parts by mass each) in separate vessels, which portions were heated to 40°C; for saponification, 10 parts by mass of the 3% by mass methanol solution of sodium hydroxide adjusted to 35°C was added to one portion, 6 parts by mass of the sodium hydroxide solution was added to the other portion, and both the mixtures were separately and simultaneously stirred at 300 rpm for 1 minute and then allowed to stand at 40°C for 40 minutes; and the separately obtained gelled materials were combined together and ground.

The measured physical properties of the obtained PVA-based polymer are shown in Table 1.

### Synthesis Example 6

Forty parts by mass of a commercial PVA resin (manufactured by JAPAN VAM & POVAL CO., LTD., JL-05E, saponification value: 80.2 mol%, viscosity of 4% by mass aqueous solution: 5.1 mPa·s) and 60 parts by mass of a commercial PVA resin (manufactured by JAPAN VAM & POVAL CO., LTD., JT-05, saponification value: 94.0 mol%, viscosity of 4% by mass aqueous solution: 5.6 mPa·s) were placed into a polyethylene bag. The bag was shaken about 100 times so that the PVA powders were mixed into a homogeneous mixture. Thus, a PVA-based polymer having a broad saponification value distribution was prepared.

The measured physical properties of the obtained PVA-based polymer are shown in Table 1.

### Synthesis Example 7

Ninety parts by mass of a commercial PVA resin (manufactured by JAPAN VAM & POVAL CO., LTD., JP-05, saponification value: 87.5 mol%, viscosity of 4% by mass aqueous solution: 5.3 mPa·s) and 10 parts by mass of a commercial PVA resin (manufactured by JAPAN VAM & POVAL CO., LTD., JT-05, saponification value: 94.0 mol%, viscosity of 4% by mass aqueous solution: 5.6 mPa·s) were placed into a polyethylene bag. The bag was shaken about 100 times so that the PVA powders were mixed into a homogeneous mixture. Thus, a PVA-based polymer having a broad saponification value distribution was prepared.

The measured physical properties of the obtained PVA-based polymer are shown in Table 1.

### Synthesis Example 8

A PVA-based polymer was obtained in the same manner as described in Comparative Synthesis Example 1 except that the mixture of the methanol solution of polyvinyl acetate and the sodium hydroxide solution was stirred at a speed of 200 rpm for 30 seconds, that is, stirring was performed under the conditions to keep the mixture less homogeneous than usual.

The measured physical properties of the obtained PVA-based polymer are shown in Table 1.

### Synthesis Example 9

Eight parts by mass of a commercial PVA resin (manufactured by JAPAN VAM & POVAL CO., LTD., JL-05E, saponification value: 80.2 mol%, viscosity of 4% by mass aqueous solution: 5.1 mPa·s) and 92 parts by mass of a commercial PVA resin (manufactured by JAPAN VAM & POVAL CO., LTD., JP-05, saponification value: 88.8 mol%, viscosity of 4% by mass aqueous solution: 5.3 mPa·s) were placed into a polyethylene bag. The bag was shaken about 100 times so that the PVA powders were mixed into a homogeneous mixture. Thus, a PVA-based polymer having a broad saponification value distribution was prepared.

The measured physical properties of the obtained PVA-based polymer are shown in Table 1.

### Synthesis Example 10

Sixty parts by mass of a commercial PVA resin (manufactured by JAPAN VAM & POVAL CO., LTD., JL-05E, saponification value: 80.2 mol%, viscosity of 4% by mass aqueous solution: 5.1 mPa·s) and 40 parts by mass of a commercial PVA resin (manufactured by JAPAN VAM & POVAL CO., LTD., JT-05, saponification value: 94.0 mol%, viscosity of 4% by mass aqueous solution: 5.6 mPa·s) were placed into a polyethylene bag. The bag was shaken about 100 times so that the PVA powders were mixed into a homogeneous mixture. Thus, a PVA-based polymer having a broad saponification value distribution was prepared.

The measured physical properties of the obtained PVA-based polymer are shown in Table 1.

### Synthesis Example 11

A PVA-based polymer was obtained in the same manner as described in Comparative Synthesis Example 1 except that a commercial polyvinyl acetate resin (manufactured by JAPAN VAM & POVAL CO., LTD., JMR-10LL, polymerization degree: 250) was used as the polyvinyl acetate resin.

The measured physical properties of the obtained PVA-based polymer are shown in Table 1.

### Synthesis Example 12

A PVA-based polymer was obtained in the same manner as described in Comparative Synthesis Example 1 except that a polyvinyl acetate resin having a polymerization degree of 800 was used as the polyvinyl acetate resin.

The measured physical properties of the obtained PVA-based polymer are shown in Table 1.

### Synthesis Examples 13 to 15

The PVA-based polymers and commercial PVA powders used in Synthesis Examples 1 to 12 were used separately to prepare the corresponding PVA-based polymers (mixtures), of which the physical properties are shown in Table 1.

The results are summarized in Table 1 below. In Table 1, "P" in parentheses means passing requirement (A) or (B), and "F" means failing requirement (A) or (B).

In Table 1, the physical properties of the commercial PVA-based polymer (manufactured by JAPAN VAM & POVAL CO., LTD., JP-05) used in Example 13 described later are also shown.

**[Table 1]**

| PVA-based polymer | Average saponification value (mol%) | Viscosity of 4% by mass aqueous solution (mPa·s) | Transparency (%) | Supernatant concentration (%) |
|---|---|---|---|---|
| Synthesis Example 1 | 88.2 | 5.2 | 18.5[P] | 0.83[P] |
| Synthesis Example 2 | 88.3 | 5.2 | 9.2[P] | 0.85[P] |
| Synthesis Example 3 | 88.3 | 5.3 | 30.0[P] | 0.76[P] |
| Synthesis Example 4 | 88.2 | 5.2 | 7.1[P] | 0.85[P] |
| Synthesis Example 5 | 88.4 | 5.3 | 5.9[P] | 0.87[P] |
| Synthesis Example 6 | 88.5 | 5.4 | 10.3[P] | 0.84[P] |
| Synthesis Example 7 | 88.2 | 5.5 | 35.4[P] | 0.71[P] |
| Synthesis Example 8 | 88.6 | 5.1 | 60.4[P] | 0.69[P] |
| Synthesis Example 9 | 88.1 | 5.3 | 99.4[F] | 0.80[P] |
| Synthesis Example 10 | 85.7 | 5.1 | 10.1[P] | 0.91[P] |
| Synthesis Example 11 | 88.3 | 3.0 | 20.7[P] | 0.85[P] |
| Synthesis Example 12 | 88.2 | 8.1 | 15.1[P] | 0.81[P] |
| Comparative Synthesis Example 1 | 88.3 | 5.3 | 99.4[F] | 0.62[F] |
| JP-05 | 88.2 | 5.3 | 99.2[F] | 0.68[P] |
| Synthesis Example 13 | 88.6 | 5.3 | 77.6[P] | 0.64[F] |
| Synthesis Example 14 | 87.8 | 5.2 | 96.6[F] | 0.71[P] |
| Synthesis Example 15 | 88.0 | 5.3 | 87.0[F] | 0.67[P] |

### Example 1

Ten parts by mass of the PVA-based polymer obtained in Synthesis Example 1 was added to 240 parts by mass of purified water, and the mixture was stirred with heating to 80°C for 1 hour to prepare a binder solution (PVA-based polymer concentration: 4% by mass). This binder solution was sprayed onto a powder mix of 400 g of lactose and 100 g of acetaminophen (average particle diameter: 40 µm) in a fluid bed granulator to produce granules [average particle diameter: 200 µm, 0.02 part by mass of the PVA-based polymer per part by mass of the powder (2.0% by mass of the PVA-based polymer per granule)].

After granulation, 475.0 g of the obtained granules and 25.0 g of a low-substituted hydroxypropyl cellulose were placed into a polyethylene bag, and the bag was shaken 100 times. To the bag, 2.5 g of magnesium stearate was added, and the bag was further shaken 30 times to give a powder for tableting (average particle diameter: 180 µm).

The powder for tableting was subjected to tableting to produce tablets. The obtained tablet was measured in terms of tablet hardness and disintegration time.

The disintegration time considerably varies with the value of tablet hardness. For this reason, comparison has to be made under the same tablet hardness conditions . To this end, tablets were produced by granulation and tableting using binder solutions at different concentrations (different proportions of the binder) (0 part by mass, 0.01 part by mass, and 0.03 part by mass of the PVA-based polymer relative to 1 part by mass of a granule), and based on the tablet hardness results, the disintegration time was calculated (estimated) at a tablet hardness of 90 N.

The results are shown in Table 2.

### Examples 2 to 16

Tablets were produced in the same manner as described in Example 1 except that granulation was performed using the commercial partially-saponified PVA (manufactured by JAPAN VAM & POVAL CO., LTD., JP-05) and the PVA-based polymers obtained in Synthesis Examples 2 to 15 instead of the PVA-based polymer obtained in Synthesis Example 1. The obtained tablets were evaluated in terms of tablet hardness and disintegration time in the same manner as described in Example 1. The results are shown in Table 2.

### Comparative Example 1

Tablets were produced in the same manner as described in Example 1 except that granulation was performed using the PVA-based polymer obtained in Comparative Synthesis Example 1 instead of the PVA-based polymer obtained in Synthesis Example 1. The obtained tablets were evaluated in terms of tablet hardness and disintegration time in the same manner as described in Example 1. The results are shown in Table 2.

**[Table 2]**

| | PVA-based polymer | Tablet hardness (N) | Disintegration time (min) |
|---|---|---|---|
| Example 1 | Synthesis Example 1 | 92 | 100 |
| Example 2 | Synthesis Example 2 | 93 | 99 |
| Example 3 | Synthesis Example 3 | 92 | 105 |
| Example 4 | Synthesis Example 4 | 95 | 99 |
| Example 5 | Synthesis Example 5 | 93 | 98 |
| Example 6 | Synthesis Example 6 | 90 | 125 |
| Example 7 | Synthesis Example 7 | 87 | 170 |
| Example 8 | Synthesis Example 8 | 87 | 190 |
| Example 9 | Synthesis Example 9 | 84 | 200 |
| Example 10 | Synthesis Example 10 | 92 | 98 |
| Example 11 | Synthesis Example 11 | 90 | 95 |
| Example 12 | Synthesis Example 12 | 93 | 195 |
| Comparative Example 1 | Comparative Synthesis Example 1 | 84 | 215 |
| Example 13 | JP-05 | 82 | 210 |
| Example 14 | Synthesis Example 13 | 86 | 200 |
| Example 15 | Synthesis Example 14 | 87 | 203 |
| Example 16 | Synthesis Example 15 | 87 | 204 |

As is clear from the results in Table 2, the tablets containing, as a binder, the PVA-based polymer which meets requirement (A) and/or (B) (in particular, at least requirement (B)) showed a shorter disintegration time as compared with the tablets containing, as a binder, the PVA-based polymer which meets neither requirement (A) nor (B), when compared under the same hardness conditions. In addition, the tablets containing, as a binder, the PVA-based polymer which meets requirement (A) and/or (B) (in particular, at least requirement (B)) generally showed a large hardness (that is, high moldability).

Surprisingly, the binder which meets requirement (A) and/or (B) (in particular, at least requirement (B)) was found to achieve a good balance between hardness and disintegrability, which originally have a trade-off relationship.

It was also found that such hardness and disintegration time can be achieved when either one of requirements (A) and (B) is met, that is, when only one of them is met regardless of whether or not the other is met.

### INDUSTRIAL APPLICABILITY

The present invention provides a novel binder. The binder is useful for various applications including the production of granulated materials from powder particles.

## Claims

1. A binder comprising a polyvinyl alcohol-based polymer, wherein the polyvinyl alcohol-based polymer has an average saponification value of 85.0 mol% to 89.0 mol% as measured according to JIS K6726 and meets requirement (A): a fluid prepared by adding 130.0 mL of 1-propanol to 100.0 g of a 5.0% by mass aqueous solution of the polyvinyl alcohol-based polymer has a transparency of 80.0% or less at 20°C.

2. A binder comprising a polyvinyl alcohol-based polymer, wherein the polyvinyl alcohol-based polymer has an average saponification value of 85.0 mol% to 89.0 mol% as measured according to JIS K6726 and meets requirement (B) : a supernatant from a fluid prepared by adding 230.0 mL of 1-propanol to 100.0 g of a 5.0% by mass aqueous solution of the polyvinyl alcohol-based polymer has a concentration of 0.65% by mass or more at 20°C.

3. The binder according to claim 1, wherein a supernatant from a fluid prepared by adding 230.0 mL of 1-propanol to 100.0 g of a 5.0% by mass aqueous solution of the polyvinyl alcohol-based polymer has a concentration of 0.3% by mass or more at 20°C.

4. The binder according to claim 2, wherein a fluid prepared by adding 130.0 mL of 1-propanol to 100.0 g of a 5.0% by mass aqueous solution of the polyvinyl alcohol-based polymer has a transparency of 95.0% or less at 20°C.

5. A binder comprising a polyvinyl alcohol-based polymer, wherein the polyvinyl alcohol-based polymer has an average saponification value of 85.0 mol% to 89.0 mol% as measured according to JIS K6726 and meets both requirement (A) : a fluid prepared by adding 130.0 mL of 1-propanol to 100.0 g of a 5.0% by mass aqueous solution of the polyvinyl alcohol-based polymer has a transparency of 80.0% or less at 20°C; and requirement (B) : a supernatant from a fluid prepared by adding 230.0 mL of 1-propanol to 100.0 g of a 5.0% by mass aqueous solution of the polyvinyl alcohol-based polymer has a concentration of 0.70% by mass or more at 20°C.

6. The binder according to claim 1 or 5, wherein the transparency specified in requirement (A) is 50.0% or less.

7. The binder according to claim 2 or 5, wherein the concentration of the supernatant specified in requirement (B) is 0.75% by mass or more.

8. The binder according to any one of claims 1 to 7, wherein a 4% by mass aqueous solution of the polyvinyl alcohol-based polymer has a viscosity of 2.0 to 10.0 mPa·s as measured according to JIS K6726.

9. The binder according to any one of claims 1 to 8, wherein the binder is used for granulation.

10. The binder according to any one of claims 1 to 9, wherein the binder is used for granulation from powder particles having an average particle diameter of 100 µm or less.

11. The binder according to any one of claims 1 to 10, wherein the binder is used for production of a granulated material having an average particle diameter of 300 µm or less.

12. A method for producing a granulated material by granulation using the binder according to any one of claims 1 to 11.

13. The method according to claim 12, wherein the method comprises a step of bringing an aqueous and/or water-based solution containing the binder into contact with powder particles.

14. A granulated material comprising the binder according to any one of claims 1 to 11 as a binder.

15. The granulated material according to claim 14, wherein the binder is present in an amount of 0.005 to 0.1 part by mass relative to 1 part by mass of powder particles.

16. A tablet comprising the granulated material according to claim 14 or 15.

17. A coated tablet comprising the tablet according to claim 16 and a polyvinyl alcohol-based polymer coating a surface of the tablet.
